(19) Europäisches Patentamt
European Patent Office
Office européen des brevets

(11) Veröffentlichungsnummer : **0 054 809**
**B1**

(12) # EUROPÄISCHE PATENTSCHRIFT

(45) Veröffentlichungstag der Patentschrift : **23.05.84**

(51) Int. Cl.³ : **C 07 C103/44**, C 07 C102/00

(21) Anmeldenummer : **81110180.7**

(22) Anmeldetag : **05.12.81**

(54) **Verfahren zur Herstellung von 5-Amino-2,4-dimethyl-acetanilid.**

(30) Priorität : **19.12.80 DE 3047946**

(43) Veröffentlichungstag der Anmeldung : **30.06.82 Patentblatt 82/26**

(45) Bekanntmachung des Hinweises auf die Patenterteilung : **23.05.84 Patentblatt 84/21**

(84) Benannte Vertragsstaaten : **CH DE FR GB IT LI SE**

(56) Entgegenhaltungen : **DE-A- 1 643 264**

(73) Patentinhaber : **BAYER AG**
**Konzernverwaltung RP Patentabteilung**
**D-5090 Leverkusen 1 Bayerwerk (DE)**

(72) Erfinder : **Behre, Horst, Dr.**
**Zur alten Linde 12**
**D-5068 Odenthal (DE)**
Erfinder : **Blank, Heinz Ulrich, Dr.**
**Am Geusfelde 35**
**D-5068 Odenthal (DE)**
Erfinder : **Seyberlich, Alfred, Dr.**
**Am Kuehnsbusch 35**
**D-5090 Leverkusen 1 (DE)**
Erfinder : **Hagedorn, Ferdinand, Dr.**
**Domblick 16**
**D-5090 Leverkusen 31 (DE)**

**EP 0 054 809 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von 5-Amino-2,4-dimethyl-acetanilid durch katalytische Hydrierung eines rohen Nitrierungsgemisches, das die verschiedenen isomeren Mononitro-2,4-dimethyl-acetanilide enthält.

Das 5-Amino-2,4-dimethyl-acetanilid ist in der Literatur beschrieben und hat beispielsweise Interesse als Zwischenprodukt für die Herstellung des Pflanzenschutzmittels 5-Acetamido-2,4-dimethyl-trifluormethansulfonanilid gefunden (DE-OS 24 06 475, DE-OS 24 12 578, US 4 013 444).

Die Herstellung des 5-Amino-2,4-dimethyl-acetanilids kann, ausgehend vom 2,4-Dimethyl-anilin (2,4-Xylidin), beispielsweise auf folgende Weise erfolgen:

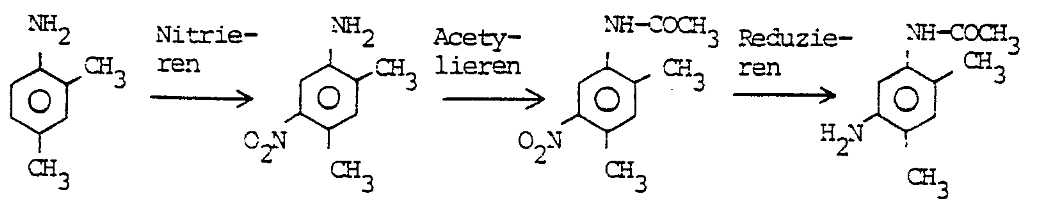


oder

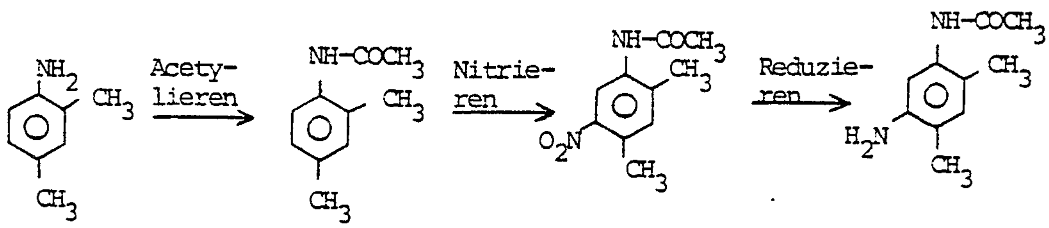


Die beiden in den Formelschemata vorkommenden Nitrierungsstufen durch Nitrierung in konzentrierter Schwefelsäure mit Salpetersäure sowie die Acetylierung sind in der Literatur beschrieben (Beilsteins Handbuch der organischen Chemie, Hauptwerk 4. Auflage 1929, Band XII, Seite 1129). Auf beiden beschriebenen Wegen wird das 5-Nitro-2,4-dimethyl-acetanilid beim Eintragen des Nitriergemisches in Wasser nicht in reiner Form ausgefällt, sondern im Gemisch mit der isomeren 3-Nitro- und 6-Nitro-Verbindung. Außerdem kann die gewünschte 5-Nitro-Verbindung in geringer Menge mit Dinitro-Verbindungen und weiteren Nebenprodukten unbekannter Struktur in wechselnden Mengen verunreinigt sein. Zur Reinigung der gewünschten 5-Nitro-Verbindung ist daher eine aufwendige Umlösung aus organischen Lösungsmitteln erforderlich, die Verluste an Material und Lösungsmitteln sowie einen erheblichen Arbeitsaufwand mit sich bringt.

Es ist weiter bekannt, das 5-Nitro-2,4-dimethyl-acetanilid mit Zinkstaub und Ammoniumchlorid in siedendem wäßrigen Ethanol (50 Gew.-%) zu 5-Amino-2,4-dimethylacetanilid zu reduzieren (J. Chem. Soc. 119, 717 bis 721 (1921)). Diese Reduktionsmethode ist jedoch für ein technischens Verfahren zu aufwendig und daher nicht geeignet.

Aus DE-OS 1 643 264 ist es bekannt, Nitroanilide in einem organischen Lösungsmittel oder Wasser und in Gegenwart einer organischen oder anorganischen Säure mit einer Dissoziationskonstante von größer als $5 \cdot 10^{-6}$ an Raney-Nickel katalytisch zu den entsprechenden Aminoverbindungen zu hydrieren. Nach den Angaben dieser DE-OS soll die Hydrierung ohne eine solche Säure nur träge oder überhaupt nicht in Gang kommen. Nach der Hydrierung und dem Abfiltrieren des Katalysators wird sodann der Reaktrionsansatz zur Trockne eingedampft, woran sich eine Umkristallisation, beispeilsweise aus Ethanol anschließen kann. Im Verfahren dieser DE-OS werden reine Ausgangsstoffe eingesetzt.

Es wurde nun ein Verfahren zur Herstellung von 5-Amino-2,4-dimethyl-acetanilid aus 5-Nitro-2,4-dimethyl-acetanilid durch katalytische Reduktion gefunden, das dadurch gekennzeichnet ist, daß man ein die isomeren Mononitro-2,4-dimethylacetanilide enthaltendes Gemisch mit einem Gehalt von 60 bis 95 Gew.-% 5-Nitro-2,4-dimethyl-acetanilid, bezogen auf das Gesamtgewicht dieses Gemisches, in einem aliphatischen Alkohol mit 1 bis 6 C-Atomen, der Wasser enthalten kann, einsetzt, nach der Hydrierung den Hydrierkontakt abtrennt und das entstandene 5-Amino-2,4-dimethyl-acetanilid aus 3 bis 20 Teilen eines Gemisches des Alkohols mit wenigstens 50 Gew.-% Wasser, bezogen auf das gesamte Lösungsmittelgemisch, pro 1 Teil des 5-Amino-2,4-dimethyl-acetanilids durch Kristallisation abtrennt.

Das erfindungemäß einzusetzende, die isomeren Mononitro-2,4-dimethyl-acetanilide enthaltende Gemisch kann beispielsweise nach einem der oben durch die Formelschemata gekennzeichneten Weg hergestellt werden. Hierbei wird beispielsweise nach Beendigung der jeweiligen Nitrierstufe das Nitriergemisch in überschüssiges Wasser gegeben, so daß das organische Material aus dem Nitriergemisch durch Fällung weitgehend isoliert wird und das erfindungsgemäß einzusetzende Gemisch der isomeren Verbindungen entsteht. Für den Fall, daß die Nitrierstufe vor der Acetylierung durchgeführt

wird, wird das bei der Nitrierung entstehende, soeben beschriebene Gemisch ohne Aufarbeitung der Acetylierung unterworfen. Die untere Grenze für den Gehalt an gewünschtem 5-Nitro-2,4-dimethyl-acetanilid liegt beispielsweise bei etwa 60 Gew.-%, bezogen auf das Gesamtgemisch, bevorzugt bei etwa 70 Gew.-%, besonders bevorzugt bei etwa 75 Gew.-%. Die obere Grenze des Gehaltes an gewünschtem 5-Nitro-2,4-dimethyl-acetanilid in dem erfindungsgemäß einzusetzenden Gemisch liegt beispielsweise bei etwa 95 Gew.-%, bezogen auf das Gesamtgewicht dieses Gemisches, bevorzugt bei etwa 93 Gew.-%, besonders bevorzugt bei etwa 90 Gew.-%. Selbstverständlich können auch Gemische, in denen das 5-Nitro-2,4-dimethyl-acetanilid in einem höheren Reinheitsgrad vorliegt, erfindungsgemäß umgesetzt werden. Selbstverständlich können weiterhin Gemische der isomeren Mononitro-2,4-dimethyl-acetanilide, die auf einem anderen als dem geschilderten Wege erhalten worden sind, erfindungsgemäß umgesetzt werden. Solche erfindungsgemäß einzusetzenden Gemische können weiterhin beispielsweise 0,1 bis 5 Gew.-%, bezogen auf die Summe der genannten 3 isomeren Mononitro-Verbindungen, an unbekannten Verbindungen enthalten. Weiterhin können solche Gemische als getrocknete oder bevorzugt als wasserfeuchte Ware, beispielsweise mit einem Wassergehalt bis zu etwa 50 Gew.-% eingesetzt werden. Sie können außerdem noch geringe Mengen an Chemikalienresten aus den vorausgegangenen und beispielhaft oben beschriebenen Umsetzungsstufen, beispielsweise Schwefelsäure und/oder anorganische Salze, wie Natriumsulfat, enthalten.

Als typisches, erfindungsgemäß einsetzbares Gemisch sei beispielsweise ein solches genannt, bei dem die isomeren Mononitro-Verbindungen ohne Nennung eventuell vorhandener weiterer Begleitsubstanzen innerhalb der folgenden Grenzen vorliegen : 75 bis 94,9 Gew.-% 5-Nitro-Verbindung, 5 bis 20 Gew.-% 3-Nitro-Verbindung und 0,1 bis 10 Gew.-% 6-Nitro-Verbindung.

Das erfindungsgemäße Verfahren wird in einem aliphatischen Alkohol mit 1 bis 6 C-Atomen durchgeführt der gegebenenfalls Wasser enthält. Hierfür seien beispielsweise Methanol, Ethanol, Propanol, Isopropanol, Butanol oder Hexanol genannt. Unter den genannten Verbindungen sind aliphatische Alkohole mit 1 bis 4 C-Atomen bevorzugt, besonders bevorzugt das Methanol. Diese Lösungsmittel können sowohl in reiner Form als auch im Gemisch mit Wasser verwendet werden. Als Mischungsverhältnisse kommen beispielsweise Gewichtsverhältnisse von Alkohol zu Wasser wie 100 : 1 bis 1 : 100, bevorzugt 100 : 1 bis 1 : 10, in Betracht.

Im erfindungsgemäßen Verfahren wird eine Konzentration des Gemisches der isomeren Mononitro-2,4-dimethylacetanilide in der zu hydrierenden Lösung bzw. Suspension von 5 bis 40 Gew.-%, bevorzugt 10 bis 35 Gew.-%, besonders bevorzugt 15 bis 30 Gew.-%, bezogen auf das Gewicht des gesamten Hydrieransatzes, eingestellt.

Das erfindungsgemäße Verfahren wird an einem Hydrierkontakt durchgeführt. Als solche seien beispielsweise Raney-Nickel-Kontake genannt, die als aktive Komponente Nickel allein oder legiert, beispielsweise mit einem oder mehreren der folgenden Metalle enthalten : Molybdän, Titan, Vanadin, Magnesium, Eisen, Kobalt und Chrom, wobei zusätzlich restliches Aluminium aus der Herstellung des Raney-Kontaktes vorhanden sein kann. Weiterhin seien beispielsweise Edelmetall-Kontakte genannt, die eines oder mehrere der Metalle Osmium, Iridium, Platin, Ruthenium, Rhodium und Palladium enthalten. Solche Edelmetall-Kontakte können mit und ohne Trägermaterial eingesetzt werden. Für den Fall, daß ein Trägermaterial eingesetzt wird, seien beispielsweise Carbonate und Sulfate der Erdalkalimetalle, wie Bariumcarbonat, Bariumsulfat, Calciumcarbonat und Calciumsulfat oder Tonerde, Aluminiumoxid, Siliciumdioxid und Kieselsäuren sowie Kohle, besonders in Form von Aktivkohle, genannt. Weiterhin seien für den Einsatz im erfindungsgemäßen Verfahren sulfidische Hydrierkontakte, wie Kobaltsulfid, genannt. Unter den erfindungsgemäß ein setzbaren Hydrierkontakten seien bevorzugt Raney-Kontakte und unter diesen bevorzugt Raney-Nickel genannt.

Die Menge des Hydrierkontaktes für das erfindungsgemäße Verfahren beträgt beispielsweise 0,5 bis 8 Gew.-%, bevorzugt 2 bis 5 Gew.-%, bezogen auf das Gesamtgewicht des Reaktionsgemisches. Diese Angaben beziehen sich auf das Trockengewicht des Kontaktes. Für den Fall, daß Hydrierkontakte, wie Raney-Kontakte, wegen ihrer pyrophoren Eigenschaften unter Wasser aufbewahrt werden müssen, muß beispielsweise bei einem 50 Gew.-% Wasser enthaltenden Raney-Kontakt die doppelte der oben angegebenen Menge eingesetzt werden. Der Wassergehalt eines solchen Kontaktes stört hierbei ebensowenig wie ein eventueller Wassergehalt des Gemisches der Mononitro-Verbindungen, da, wie bereits dargelegt, das erfindungsgemäße Verfahren gegebenenfalls und in einer bevorzugten Ausführungsform in einem wasserhaltigen Alkohol durchgeführt werden kann. Das erfindungsgemäße Verfahren kann selbstverständlich auch mit einem höheren als dem angegebenen Kontakteinsatz durchgeführt werden. Hierbei ergeben sich jedoch keine besonderen Vorteile, so daß dies aus wirtschaftlichen Überlegungen heraus weniger bevorzugt ist.

Der Hydrierkontakt kann bei dem erfindungsgemäßen Verfahren mehrfach verwendet werden. Hierbei bleibt die Aktivität des Hydrierkontaktes weitgehend oder sogar vollständig erhalten. Dies ist außerordentlich überraschend, da unreine Ausgangssubstanzen, wie die erfindungsgemäß einzusetzenden Gemische, insbesondere wenn sie noch unbekannte Verbindungen enthalten, stets die Gefahr der Kontaktvergiftung in sich bergen. Diese dem Fachmann von aktiven Hydrierkontakten her allgemein bekannte Tatsache wird im erfindungsgemäßen Verfahren nicht beobachtet. Für den Fall, daß eine größere Anzahl von Reaktionsansätzen unter Wiederverwendung des Hydrierkontaktes ausgeführt werden, beispielsweise mehr als 10 oder 20, kann es jedoch günstig sein, vorsorglich nach jedem Ansatz

eine kleine Menge des zurückgewonnenen Hydrierkontaktes zu ersetzen. Als eine solche kleine auszuschleusende Menge sei beispielsweise 0,01 bis 4 Gew.-%, bevorzugt 0,05 bis 2 Gew.-%, bezogen auf das Gewicht des wiedergewonnenen Kontaktes, genannt. Diese äußerst geringen Kontaktverbräuche stellen einen besonders günstigen wirtschaftlichen Aspekt des erfindungsgemäßen Verfahrens dar.

Die Hydrierung kann beispielsweise bei einer Temperatur von 20 bis 200 °C, bevorzugt 40 bis 120 °C, und einem Wasserstoffdruck von beispielsweise 1 bis 200 bar, bevorzugt 3 bis 50 bar, besonders bevorzugt 5 bis 25 bar, durchgeführt werden.

Es ist ein wesentliches Merkmal des erfindungsgemäßen Verfahrens, daß das durch Hydrierung gebildete 5-Amino-2,4-dimethyl-acetanilid durch Kristallisation aus einem Gemisch des aliphatischen Alkohols mit 1 bis 6 C-Atomen und Wasser von den unerwünschten Begleitstoffen abgetrennt wird. Dies ist überraschend, da es bekannt ist, daß Kristallisationen organischer Verbindungen aus einem wasserhaltigen Gemisch, insbesondere aus einem wasserreichen Gemisch, oftmals zu Mitfällungen führen. Weiterhin überraschend ist die Tatsache, daß unter den nicht gewünschten Isomeren auch das sehr schlecht lösliche 6-Amino-Isomere in der Kristallisations-Mutterlauge verbleibt und die Gewinnung eines reinen 5-Amino-2,4-dimethyl-acetanilids mit einer Reinheit von 99,5 bis 99,9 Gew.-%, bezogen auf das gesamte kristallin erhaltene Reaktionsprodukt, ermöglicht. In dem zur Kristallisation verwendeten Gemisch aus dem Alkohol und Wasser liegt das Wasser in einer Menge von wenigstens etwa 50 Gew.-%, bezogen auf das gesamte Lösungsmittelgemisch, vor, beispielsweise in einer Menge von etwa 50 bis etwa 95 Gew.-%.

Beispielhaft sei für die bevorzugt einzusetzenden niederen aliphatischen Alkohole, insbesondere für das Methanol, ein Wasser/Alkohol-Gewichtsverhältnis im Bereich von 2 : 1 bis 20 : 1, bevorzugt 3 : 1 bis 10 : 1, angegeben. Für die besonders bevorzugt einzusetzenden rohen Mononitro-2,4-dimethyl-acetanilid-Gemische, die oben beschrieben wurden, werden beispielsweise gute Ergebnisse erhalten, wenn man das 5-Amino-2,4-dimethyl-acetanilid aus einem Wasser/Methanol-Gemisch im Gewichtsverhältnis von etwa 3 : 1 bis 10 : 1 kristallisiert. Hierfür sind pro 1 Gew.-Teil 5-Amino-2,4-dimethyl-acetanilid etwa 3 bis 20, bevorzugt 5 bis 15 Gew.-Teile eines solchen Wasser/Methanol-Gemisches erforderlich.

Zur Einstellung eines solchen günstigen Wasser/Alkhol-Gewichtsverhältnisses in Verbindung mit dem oben beschriebenen günstigen Substrat/Alkohol-Gemisch für die Hydrierung kann es erforderlich sein, nach der Hydrierung und nach der Abtrennung des Hydrierkontaktes, beispielsweise durch Filtration oder Zentrifugieren, einen Teil des im Hydrierungsgemisch vorhandenen Alkohols, beispielsweise Methanol, durch Destillation zu entfernen, wobei für den Fall, daß das Hydrierungsgemisch Wasser enthält, bei dieser Destillation auch ein Teil dieses Wassers abdestillieren kann. Als Destillationsrückstand wird hierbei eine konzentrierte alkoholische oder alkoholisch-wäßrige, beispielsweise methanolische oder methanolisch-wäßrige, Lösung oder Suspension eines Monoamino-2,4-Dimethyl-acetanilid-Gemisches erhalten, die direkt durch Kühlen zur Kristallisation oder weiteren Kristallisation gebracht werden kann oder nach vorangegangener Zugabe von Wasser oder gegebenenfalls von weiterem Alkohol, beispielsweise Methanol, zur Einstellung der oben ausgeführten optimalen Substrat- und Alkohol-abhängigen Alkoholmenge zur Kristallisation gebracht wird. Die Zugabe von Wasser (oder seltener von einem Alkohol) kann jedoch auch in das vom Hydrierkontakt befreite Hydrierungsgemisch erfolgen, bevor eine destillative Abtrennung des Alkohols, oder in selteneren Fällen des Wassers, erfolgt. Dieser letztere Fall kann beispielsweise vorteilhaft sein, wenn die Hydrierung in Methanol oder in einem wäßrigen Methanol mit beispielsweise 10 bis 50 Gew.-% Wassergehalt, bezogen auf das Methanol/Wasser-Gemisch, durchgeführt wurde. In einem solchen Fall kann schließlich auch in noch einer weiteren Variante das Wasser (oder in selteneren Fällen der Alkohol) während einer solchen Destillation zugesetzt werden.

Weiterhin ist es im Rahmen des erfindungsgemäßen Verfahrens möglich, bereits zur Hydrierung ein solches Wasser/Alkohol-Gewichtsverhältnis einzustellen, wie es für die nachfolgende Kristallisation der gewünschten, durch Hydrierung erhaltenen Amino-Verbindung vorgesehen ist.

Zur Kristallisation des 5-Amino-2,4-dimethyl-acetanilids wird eine Temperatur von beispielsweise 0 bis 60 °C, bevorzugt 5 bis 30 °C, eingestellt.

Das erfindungsgemäße Verfahren besitzt folgende wesentliche Vorteile gegenüber den aus der Literatur bekannten Verfahren :

1. Einsatz eines technischen Mononitro-2,4-dimethyl-acetanilid-Isomerengemisches, wodurch eine aufwendige Reinigung des gewünschten 5-Nitro-2,4-dimethyl-acetanilids in einer Reinigungsoperation, beispielsweise durch Umkristallisation aus organischen Lösungsmitteln, die mit Ausbeuteverlusten verbunden ist, vermieden werden kann.

2. Isolierung der 5-Amino-2,4-dimethyl-acetanilids in einer sehr einfachen und wirtschaftlichen Weise durch Benutzung stark wasserhaltiger Kristallisationsgemische. Überraschenderweise werden hierbei hohe Reinheiten erzielt, obwohl das Vorliegen von schwerlöslichen Verbindungen im Reaktionsgemisch, wie des 6-Amino-Isomeren und etwa unveränderten Nitro-Verbindungen, die Notwendigkeit von organischen Kristallisationsmedien und eine mehrfache Durchführung der Kristallisation erwarten ließen.

## Beispiel 1

In einem 2,7-l-Hydrierautoklaven werden 200 g Mononitro-2,4-dimethyl-acetanilid-Isomerengemisch

folgender Zusammensetzung :

77 Gew.-% 5-Nitro-2,4-dimethyl-acetanilid
14 Gew.-% 3-Nitro-2,4-dimethyl-acetanilid
6 Gew.-% 6-Nitro-2,4-dimethyl-acetanilid
2,5 Gew.-% unbekannte Verunreinigungen
0,5 Gew.-% Wasser

sowie 635 g Methanol und 40 g Raney-Nickel 100 %ig eingesetzt.

Die Reaktionsmischung wird bei 80 °C und einem Wasserstoffdruck von 20 bar in 30 Minuten hydriert und nach beendeter Wasserstoff-Aufnahme 30 Minuten bei 80 °C nachgerührt. Der Katalysator wird heiß abfiltriert und mit 80 g Methanol gewaschen. Aus dem Filtrat werden bei Normaldruck über eine Kolonne 475 g Methanol abdestilliert. Die heiße Destillationsrückstandslösung läßt man unter Rühren in 1 500 g Wasser einlaufen. Die Reaktionmischung wird auf 20 °C kalt gerührt und 1 Stunde bei dieser Temperatur nachgerührt. Das ausgefallene Produkt wird abfiltriert, 2-mal mit je 200 g kaltem Wasser gewaschen und im Vakuum bei 60 °C getrocknet. Ausbeute 110 g trocknes Produkt mit Fp. 162 bis 163 °C, entsprechend einer Ausbeute von 84 % der theoretischen Ausbeute, bezogen auf den Gehalt an 5-Nitro-2,4-dimethyl-acetanilid in dem eingesetzten Mononitro-2,4-dimethyl-acetanilid-Isomerengemisch.

In dem isolierten Produkt wurden mittels Diazotierung und Dünnschichtchromatographie folgende Gehalte bestimmt :

99,5 Gew.-% 5-Amino-2,4-dimethyl-acetanilid
0,2 Gew.-% 3-Amino-2,4-dimethyl-acetanilid
0,1 Gew.-% 6-Amino-2,4-dimethyl-acetanilid
0,2 Gew.-% unbekannte Verbindungen.

Beispiel 2

Eine wie im Beispiel 1 durchgeführte Hydrierung, aber bei einer Temperatur von 70 °C und einem Wasserstoffdruck von 10 bar, führt zu einer Ausbeute von 86 % der theoretischen Ausbeute, bezogen auf den Gehalt an 5-Nitro-2,4-dimethyl-acetanilid in dem eingesetzten Mononitro-2,4-dimethyl-acetanilid-Isomerengemisch.

Beispiel 3

In einem 2,7-l-Hydrierautoklaven werden 230 g wasserfeuchtes Mononitro-2,4-dimethyl-acetanilid-Isomerengemisch (entsprechend 160 g trockenes Produkt) folgender Zusammensetzung, bezogen auf Trockensubstanz :

90,2 Gew.-% 5-Nitro-2,4-dimethyl-acetanilid
9,5 Gew.-% 3-Nitro-2,4-dimethyl-acetanilid
0,3 Gew.-% 6-Nitro-2,4-dimethyl-acetanilid

sowie 792 g Methanol und 30 g Raney-Nickel 100 %ig eingesetzt. Die Reaktionsmischung wird bei 80 °C und einem Wasserstoffdruck von 10 bar in 1 Stunde hydriert und nach beendeter Wasserstoffaufnahme 30 Minuten bei 80 °C nachgerührt. Der Katalysator wird heiß abfiltriert und mit 40 g Methanol gewaschen. Aus dem Filtrat werden bei Normaldruck über eine Kolonne 600 g Methanol abdestilliert. Die heiße Destillationsrückstandslösung läßt man unter Rühren in 1 000 g Wasser einlaufen. Die Reaktionsmischung wird auf 20 °C unter Rühren abgekühlt, das ausgefallene Produkt filtriert, nacheinander mit 150 g Wasser von 20 °C und 150 g Wasser von 0 °C gewaschen und im Vakuum bei 60 °C getrocknet. Ausbeute 107 g trockenes Produkt mit Fp. 165 bis 166 °C, entsprechend einer Ausbeute von 86 % der theoretischen Ausbeute, bezogen auf den Gehalt an 5-Nitro-2,4-dimethyl-acetanilid in dem eingesetzten Mononitro-2,4-dimethyl-acetanilid-Isomerengemisch.

In dem isolierten Produkt wurden mittels Diazotierung und Dünnschichtchromatographie folgende Gehalte bestimmt :

99,9 Gew.-% 5-Amino-2,4-dimethyl-acetanilid
0,1 Gew.-% unbekannte Verbindung

Beispiel 3a

Herstellung des Mononitro-2,4-dimethyl-acetanilid-Isomerengemisches

In einem 1-l-Vierhalskolben mit Rührer, Kühler, Tropftrichter und Innenthermometer werden 300 g (5,0 Mol) Eisessig vorgelegt. Bei 20 °C werden 141 g Mononitro-2,4-dimethyl-anilin-Isomerengemisch (hergestellt durch Nitrierung von 121 g ≙ 1,0 Mol. 2,4-Dimethyl-anilin) folgender Zusammensetzung :

90,2 Gew.-% 5 Nitro-2,4-dimethyl-anilin
9,5 Gew.-% 3-Nitro-2,4-dimethyl-anilin
0,3 Gew.-% 6-Nitro-2,4-dimethyl-anilin

eingetragen. Unter Kühlung werden in 1 Stunde 122 g (1,2 Mol) Acetanhydrid eingetropft, wobei die Temperatur nicht über 55 °C ansteigen soll. Die Reaktionsmischung wird dann zum Sieden erhitzt und 1 Stunde unter Rückfluß erhitzt. Anschließend werden 300 g Eisessig bei Normaldruck abdestilliert. Die ca. 120 °C heiße Reaktionslösung läßt man unter Rühren in eine Vorlage mit 1 500 g Wasser von 35 °C einlaufen, wobei die Temperatur nicht über 60 °C ansteigen soll. Die Reaktionsmischung wird auf 20 °C unter Rühren abgekühlt, das ausgefallene Produkt filtriert, mit Wasser Essigsäure-frei gewaschen und gut trocken gesaugt. Ausbeute etwa 230 g feuchtes Produkt mit der oben angegebenen relativen Zusammensetzung.

Beispiel 4

Eine wie im Beispiel 3 — aber mit 15-maliger Wiederverwendung des gebrauchten Raney-Nickel-Katalysators — durchgeführte Hydrierung führte zu einer Ausbeute von 84 % der Theorie. Eine Verlängerung der Hydrierzeit war nicht erforderlich.

Beispiel 5

Eine wie in Beispiel 3 — aber mit 400 g Methanol und 400 g Wasser bei 60 °C und einem Wasserstoffdruck von 15 bar — durchgeführte Hydrierung führte nach Abdestillieren von 300 g Methanol und Zugabe von 600 g Wasser zu einer Ausbeute von 86 % der theoretischen Ausbeute.

Beispiel 6

Eine wie in Beispiel 1 — aber in 235 g Methanol und 1 200 g Wasser bei 60 °C und bei einem Wasserstoffdruck von 10 bar — durchgeführte Hydrierung führte ohne Abdestillieren von Methanol und ohne Zugabe von Wasser zu einer Ausbeute von 90 %, bezogen auf den Gehalt von 5-Nitro-2,4-dimethylacetanilid in dem eingesetzten Mononitro-2,4-dimethylacetanilid-Isomerengemisch. In dem isolierten Produkt wurden mittels Diazotierung und Dünnschichtchromatographie folgende Gehalte bestimmt :

99,7 Gew.-% 5-Amino-2,4-dimethylacetanilid
0,2 Gew.-% 3-Amino-2,4-dimethylacetanilid
0,0 Gew.-% 6-Amino-2,4-dimethylacetanilid
0,1 Gew.-% unbekannte Verbindungen.

**Ansprüche**

1. Verfahren zur Herstellung von 5-Amino-2,4-dimethyl-acetanilid aus 5-Nitro-2,4-dimethyl- acetanilid durch katalytische Hydrierung, dadurch gekennzeichnet, daß man ein die isomeren Mononitro-2,4-dimethyl-acetanilide enthaltendes Gemisch mit einem Gehalt von 60 bis 95 Gew.-% 5-Nitro-2,4-dimethyl-acetanilid, bezogen auf das Gesamtgewicht dieses Gemisches, in einem aliphatischen Alkohol mit 1 bis 6 C-Atomen, der Wasser enthalten kann, einsetzt, nach der Hydrierung den Hydrierkontakt abtrennt und das entstandene 5-Amino-2,4-dimethyl-acetanilid aus 3 bis 20 Teilen eines Gemisches des Alkohols mit wenigstens 50 Gew.-% Wasser, bezogen auf das gesamte Lösungsmittelgemisch, pro 1 Teil des 5-Amino-2,4-dimethylacetanilids durch Kristallisation abtrennt.

2. Verfahren nach Anspruch 1, dadurch gekennzeichnet, daß das Gemisch mindestens 70 Gew.-% an 5-Nitro-2,4-dimethyl-acetanilid enthält.

3. Verfahren nach Ansprüchen 1 und 2, dadurch gekennzeichnet, daß das Gemisch 75 bis 94,9 Gew.-% 5-Nitro-, 5 bis 20 Gew.-% 3-Nitro- und 0,1 bis 10 Gew.-% 6-Nitro-2,4-dimethyl-acetanilid, bezogen auf die Summe der genannten Nitroverbindungen, enthält.

4. Verfahren nach Ansprüchen 1 bis 3, dadurch gekennzeichnet, daß ein aliphatischer Alkohol und 1 bis 4 C-Atomen eingesetzt wird.

5. Verfahren nach Ansprüchen 1 bis 4, dadurch gekennzeichnet, daß die Umsetzung in Methanol oder Methanol/Wasser durchgeführt wird, wobei Methanol und Wasser im Gewichtsverhältnis von 100 : 1 bis 1 : 100 stehen.

6. Verfahren nach Ansprüchen 1 bis 5, dadurch gekennzeichnet, daß der Hydrierkontakt in einer Menge von 0,5 bis 8 Gew.-%, bezogen auf die Gesamtmenge des Reaktionsgemisches, eingesetzt wird.

7. Verfahren nach Ansprüchen 1 bis 6, dadurch gekennzeichnet, daß die Kristallisation des 5-Amino-2,4-dimethyl-acetanilids aus Wasser/Alkohol-Gemischen mit einem Gewichtsverhältnis von Wasser zu

Alkohol wie 2 : 1 bis 20 : 1 durchgeführt wird.

8. Verfahren nach Ansprüchen 1 bis 7, dadurch gekennzeichnet, daß die Kristallisation aus einem Wasser/Methanol-Gemisch mit einem Gewichtsverhältnis von Wasser zu Methanol wie 3 : 1 bis 10 : 1 und einem Verhältnis von 3 bis 20 Gew.-Teilen eines solchen Wasser/Methanol-Gemisches zu einem Gew.-Teil isolierter 5-Amino-Verbindung durchgeführt wird.

9. Verfahren nach Ansprüchen 1 bis 8, dadurch gekennzeichnet, daß die Kristallisation bei einer Temperatur von 0 bis 60 °C durchgeführt wird.

**Claims**

1. Process for the preparation of 5-amino-2,4-dimethylacetanilide from 5-nitro-2,4-dimethylacetanilide by catalytic hydrogenation, characterised in that a mixture containing the isomeric mononitro-2,4-dimethylacetanilides, with a content of 60 to 95 % by weight of 5-nitro-2,4-dimethylacetanilide, relative to the total weight of this mixture, in an aliphatic alcohol with 1 to 6 C atoms which can contain water, is used, the hydrogenation catalyst is separated off after hydrogenation and the 5-amino-2,4-dimethylacetanilide formed is separated by crystallisation from 3 to 20 parts of a mixture of the alcohol and at least 50 % by weight of water, relative to the total solvent mixture, per part of 5-amino-2,4-dimethyl-acetanilide.

2. Process according to Claim 1, characterised in that the mixture contains at least 70 % by weight of 5-nitro-2,4-dimethylacetanilide.

3. Process according to Claims 1 and 2, characterised in that the mixture contains 75 to 94.9 % by weight of 5-nitro-2,4-dimethylacetanilide, 5 to 20 % by weight of 3-nitro-2,4-dimethylacetanilide and 0.1 to 10 % by weight of 6-nitro-2,4-dimethylacetanilide, relative to the sum of the nitro compounds mentioned.

4. Process according to Claims 1 to 3, characterised in that a lower aliphatic alcohol and 1 to 4 C atoms is used.

5. Process according to Claims 1 to 4, characterised in that the reaction is carried out in methanol or methanol/water, methanol and water being present in the ratio by weight of from 100 : 1 to 1 : 100.

6. Process according to Claims 1 to 5, characterised in that the hydrogenation catalyst is employed in a quantity of from 0.5 to 8 % by weight, relative to the total amount of the reaction mixture.

7. Process according to Claims 1 to 6, characterised in that the crystallisation of the 5-amino-2,4-dimethylacetanilide is carried out from water/alcohol mixtures with a ratio by weight of water to alcohol of 2 : 1 to 20 : 1.

8. Process according to Claims 1 to 7, characterised in that the crystallisation is carried out from a water/methanol mixture with a ratio by weight of water to methanol of 3 : 1 to 10 : 1 and a proportion of 3 to 20 parts by weight of such a water/methanol mixture to one part by weight of isolated 5-amino-compound.

9. Process according to Claims 1 to 8, characterised in that the crystallisation is carried out at a temperature of from 0 to 60 °C.

**Revendications**

1. Procédé pour la fabrication de 5-amino-2,4-diméthylacétanilide à partir du 5-nitro-2,4-diméthylacétanilide à partir du 5-nitro-2,4-diméthylacétanilide par hydrogénation catalytique, caractérisé en ce que l'on utilise un mélange contenant les mononitro-2,4-diméthylacétanilides isomères à une teneur de 60 à 95 % en poids de 5-nitro-2,4-diméthylacétanilide, par rapport au poids total de ce mélange, dans un alcool aliphatique en $C_1$-$C_6$ qui peut contenir de l'eau, on sépare après l'hydrogénation le catalyseur d'hydrogénation et on sépare par cristallisation le 5-amino-2,4-diméthylacétanilide formé dans 3 à 20 parties d'un mélange de l'alcool avec au moins 50 % en poids d'eau, par rapport au mélange solvant total, pour 1 partie du 5-amino-2,4-diméthylacétanilide.

2. Procédé selon la revendication 1, caractérisé en ce que le mélange contient au moins 70 % en poids de 5-nitro-2,4-diméthylacétanilide.

3. Procédé selon les revendications 1 et 2, caractérisé en ce que le mélange contient 75 à 94 % en poids de 5-nitro-, 5 à 20 % en poids de 3-nitro et 0,1 à 10 % en poids de 6-nitro-2,4-diméthylacétanilide, par rapport à la somme des composés nitro mentionnés.

4. Procédé selon les revendications 1 à 3, caractérisé en ce que l'on utilise un alcool aliphatique en $C_1$-$C_4$.

5. Procédé selon les revendications 1 à 4, caractérisé en ce que la réaction est effectuée dans le méthanol ou le méthanol aqueux, le méthanol et l'eau étant dans un rapport de 100 : 1 à 1 : 100 en poids.

6. Procédé selon les revendications 1 à 5, caractérisé en ce que l'on utilise le catalyseur d'hydrogénation en quantité de 0,5 à 8 % en poids, par rapport à la quantité totale du mélange de réaction.

7. Procédé selon les revendications 1 à 6, caractérisé en ce que la cristallisation du 5-amino-2,4-diméthylacétanilide est effectuée dans des mélanges eau/alcool dans un rapport pondéral eau-alcool de 2 : 1 à 20 : 1.

8. Procédé selon les revendications 1 à 7, caractérisé en ce que la cristallisation est effectuée dans un mélange eau/méthanol dans un rapport pondéral eau/méthanol de 3 : 1 à 10 : 1 et un rapport de 3 à 20 parties de ce mélange eau/méthanol pour 1 partie en poids de composé 5-amino isolé.

9. Procédé selon les revendications 1 à 8, caractérisé en ce que la cristallisation est effectuée à une température de 0 à 60 °C.